(19) Europäisches Patentamt
European Patent Office
Office européen des brevets

(11) **EP 3 515 286 B1**

(12) **EUROPEAN PATENT SPECIFICATION**

(45) Date of publication and mention
of the grant of the patent:
**08.04.2020   Bulletin 2020/15**

(51) Int Cl.:
*A61B 5/00* (2006.01)     *A61B 5/0225* (2006.01)
*A61B 5/024* (2006.01)     *A61B 5/01* (2006.01)
*H01L 41/193* (2006.01)

(21) Application number: **17765123.9**

(22) Date of filing: **01.09.2017**

(86) International application number:
**PCT/EP2017/072033**

(87) International publication number:
**WO 2018/054665 (29.03.2018 Gazette 2018/13)**

(54) **SENSOR POSITIONING USING ELECTROACTIVE POLYMERS**

SENSORPOSITIONIERUNG MITTELS ELEKTROAKTIVER POLYMERE

POSITIONNEMENT DE CAPTEUR PAR POLYMÈRES ÉLECTROACTIFS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.09.2016   EP 16190026**

(43) Date of publication of application:
**31.07.2019   Bulletin 2019/31**

(73) Proprietor: **Koninklijke Philips N.V.
5656 AG Eindhoven (NL)**

(72) Inventors:
• **HILGERS, Achim
5656 AE Eindhoven (NL)**
• **VAN DEN ENDE, Daan, Anton
5656 AE Eindhoven (NL)**

(74) Representative: **Marsman, Albert Willem
Philips Intellectual Property & Standards
High Tech Campus 5
5656 AE Eindhoven (NL)**

(56) References cited:
WO-A1-2016/096391     WO-A1-2016/193131
WO-A1-2017/036695     US-A1- 2002 130 673
US-B1- 6 545 384

• SHAHINPOOR M ET AL: "Ionic Polymer Metal Composites IPMC As Biomimetic Sensors and Actuators", OPTOMECHATRONIC MICRO/NANO DEVICES AND COMPONENTS III : 8 - 10 OCTOBER 2007, LAUSANNE, SWITZERLAND; [PROCEEDINGS OF SPIE , ISSN 0277-786X], SPIE, BELLINGHAM, WASH, 1 January 1998 (1998-01-01), pages 1-17, XP002478345, DOI: 10.1117/12.316870 ISBN: 978-1-62841-730-2

**Description**

FIELD OF THE INVENTION

This invention relates to an apparatus and method for measuring a physiological parameter, and in particular an apparatus and method adapted to optimise a positioning of a sensing surface against a human tissue receiving surface.

BACKGROUND OF THE INVENTION

Measurement of vital signs is of central importance in the monitoring of mental health and in detecting potential medical pathologies. Vital signs are measures of the human body's most basic functions. In addition to body temperature and respiration rate (breathing rate), perhaps the most critical of vital signs routinely monitored by medical professionals are the pulse rate and the blood pressure. These may be measured in a medical setting, at home, at the site of an emergency, or elsewhere. Ability to measure these with reliability and accuracy, regardless of the setting and circumstances, therefore is of great importance.

In addition to vital signs, other more complex physiological body parameters such as for example EEG (electroencephalography) - related to the brain - ECG (electrocardiogram) - related to the heart - and $S_PO_2$ (peripheral oxygen saturation) are often required in the professional medical domain in order to obtain more detailed information about the human body and its functionality.

A range of means exist for obtaining measures of these various vital signs and physiological parameters. Typically these are derived from measurements performed in one or more of the electrical, optical/visual, mechanical or acoustic domains. Often, measurement principles may be combined to improve the precision and quality of the obtained result. Performing these measurements is achieved using a range of different dedicated and often specialised sensors or measurement devices.

One particularly common type of device makes use of the photoplethysmographic (PPG) method. This is an electro-optical technique for measuring the cardiovascular pulse wave which is exhibited throughout the body. It is caused by the periodic pulsation in arterial blood volume, and is measured by the consequential change in optical absorption which this induces. PPG measurement devices typically consist of a light source (usually an infrared LED), a photodiode detector for detecting reflected or transmitted light, and a signal recovery/processor/display system. PPG is a popular technique, since it allows measurement of a number of vital signs to be obtained using a single device, including pulse rate, peripheral perfusion, pulsatile arterial blood volume, non-pulsatile arterial blood volume, and venous and capillary blood volume or flow.

Another very common approach implemented in a range of devices is the tonometry method. Tonometry allows measurement of blood pressure, and does not involve application of optical stimuli. The method is based on application of a controlled force orthogonally to the wall of a superficial artery, pressing it against an adjacent bone. This creates a local compression of the artery, and a force sensor is then employed to measure the pressure at contact. The contact is maintained throughout the heart cycle, and for best results the applied (occluding) force should change in parallel with the changing phase of the pulse-pressure wave.

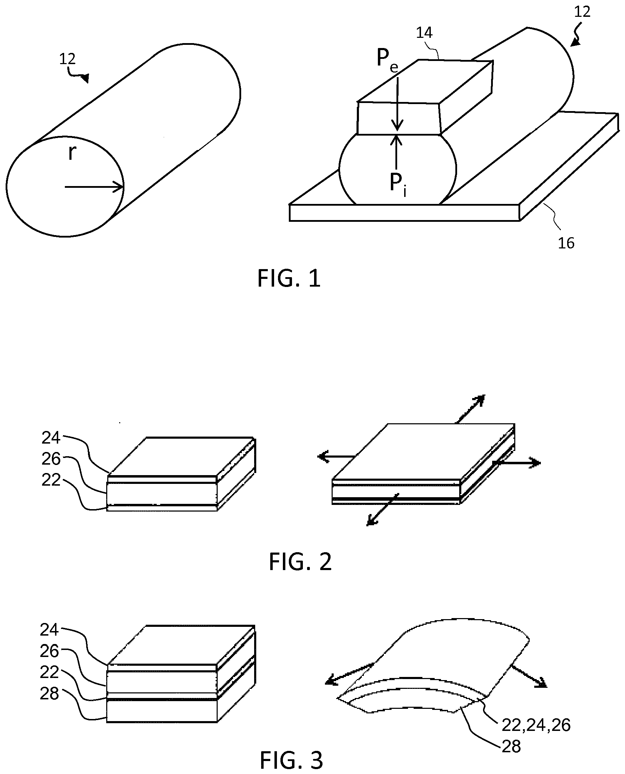

Figure 1 illustrates the principle of arterial applanation tonometry, in which an artery 12 is modelled as a cylindrical thin wall tube. In the measurement position, the pressure sensor 14 exerts a pressure on the artery 12, partially flattening the artery. The transmural pressure ($P_t$) of the artery is equal to the difference between the internal pressure ($P_i$) of the artery and the external pressure ($P_e$) being exerted upon the artery ($P_t = P_i - P_e$). According to Laplace's law, the wall tension (T) of a cylindrical thin wall tube is determined by the transmural pressure ($P_t$), the thickness ($\mu$) of the wall and the radius of the wall curvature (r):

$$T = \frac{P_t r}{\mu} \qquad (1)$$

The transmural pressure may therefore be expressed:

$$P_t = P_i - P_e = \frac{\mu T}{r} \qquad (2)$$

When the pressure sensor 14 exerts pressure on the artery wall, the artery 12 is partially flattened and consequently the radius of curvature of the artery wall (r) may be approximated as effectively infinite. As can be seen from equation (2), as r tends to infinity, so $P_t$ tends toward zero, and therefore, the internal pressure of the artery may be approximated as equal to the external pressure measured by the pressure sensor in the case that the artery is flattened.

Figure 1 shows the artery 12 flattened against a bone 16. The artery is only partially flattened; it is neither necessary nor desirable to fully close the lumen of the artery. Once in this partially flattened state, the external pressure exerted on the artery wall by the pressure sensor 14 may be taken as approximately equal to the internal pressure of the artery. Therefore, the output of the pressure sensor 14 may be taken as reflecting the blood pressure of the subject.

Depending upon the measurement approach and the parameter being measured, a physiological parameter sensor may be mounted to the user in a number of different configurations. In particular, the position of the mounted physiological parameter sensor relative to the subject may vary according to the parameter to be measured, the type of physiological parameter sensor, and/or the circumstances in which physiological sensing takes place. In some cases, the physiological parameter sensor should be in contact with the user's body. In other cases, the physiological parameter sensor should be separated from the subject's body.

A significant difficulty with respect to such sensors is ensuring that the contact pressure or separation between the physiological parameter sensor and the user's body is maintained at a constant level, since the signal obtained during physiological sensing is affected by the positioning of the sensor with respect to the user. So-called 'motion artefacts' can be created when the sensor is moved with respect to the skin, and this can lead to significant inaccuracies in the obtained measurement results.

For instance, in PPG monitoring, a steady distance between the light sensor and the skin is desired for optimal stability of the sensor signal. For ultrasound transducer patches, good contact with the skin is imperative for high quality images. Similarly, the electrodes of ECG monitoring devices are sensitive to contact pressure.

In the case of tonometry, the positioning of the tonometer over the centre of the artery is also very important. The difference between correct and incorrect placements may be in the order of millimetres. If the sensor placement is incorrect, it may lead to nonlinearity in the obtained blood pressure measurement. Tonometry is also highly sensitive to motion, so static positioning is important. Additionally, the pressure applied to the artery wall in tonometry methods must be very precisely controlled, since too little can lead to inaccurate measurement, and too much may close the artery all-together, leading to a risk of ischemia.

In many cases therefore, sensing devices would benefit from means for reliably and precisely preventing or remedying the problems caused by accidental movement. A preferred means would be one that allowed reliable and precise re-adjustment of the sensor position relative to the skin, in real-time, to enable compensation for potential motion artefacts.

It has been proposed to incorporate one or more mechatronic actuators into a sensing device, to enable compensation for accidental movement. Such approaches also confer the benefit of enabling certain stimulus pressures or forces to be exerted to the skin or to the sensor, to assist or improve in the measurement process itself. Certain pressures might be exerted to points on the body to stimulate a certain interaction or reaction, which may then be sensed, either to provide a direct measure of some physiological parameter, or to provide a proxy measure of a parameter. For instance, in the case of tonometry, incorporation of actuators enables the initial flattening of the artery to be performed mechantronically, as opposed to manually.

Furthermore, in any case where electrical, optical/visual or mechanical signals are required to be measured (as for example in the case of blood pressure, heartbeat or $S_pO_2$), the force and positioning with which sensing elements are applied to the skin must in many cases be performed with great precision, in order to avoid erroneous results.

US 2008/0033275 discloses use of electromechanical actuators to enable repositioning of a sensor module relative to a subject. While such an approach provides some benefit in terms of compensating for motion artefacts, it carries a number of significant drawbacks. In particular, electroactive actuators generally offer limited precision in the degree of control available over their movement and positioning, since they generally lack any means for providing intrinsic feedback as to their extent of actuation. Another difficulty, especially for tonometry applications, is that the position of the electromechanical actuator and the pressure sensor (14 in Figure 1) is not identical and coincident; the two are laterally displaced from one another. As a result, accurate measurement of the artery pressure $P_i$ may be compromised, or flattening of the artery may not be completely uniform. Electromechanical solutions are also unsatisfactory due to generally large form factor, elevated noise levels, and high energy consumption.

WO 2016/096391 A1 discloses a similar sensor apparatus, but requires an additional strain sensor for detecting movement of the sensing surface, distinct from the electroactive polymer structure. Said sensor can be integrated with the function of the physiological sensor, but not with the actuator. Hence, said apparatus does not use the sensing abilities of the electroactive polymer structure of the actuator.

It has been proposed to incorporate electroactive polymer based actuators into physiological sensing devices to enable manipulation of one or more measuring components against a subject's body. However, while known approaches offer a number of improvements (including form factor, noise, energy consumption, reliability and speed of response) they still maintain the same difficulty of not offering any intrinsic feedback capability, to enable the precise positioning and actuation extent of the actuator to be known in real-time. Additionally, devices incorporating such actuators still require separate, dedicated force or pressure sensors to be provided to enable any mechanical physiological parameters (such as blood pressure) to be ascertained and/or to provide feedback on the actuator position or actuation extent). Displaced actuation and sensing elements again limits accuracy of the device, especially in the case of tonometry, where precise positioning and applied pressure is very important. A displaced sensor may compromise this and reduce the effectiveness of motion artefact compensation.

There is a need therefore for a sensing apparatus capable of adjusting the positioning or application pressure of a physiological sensor against a human tissue surface at least to compensate for motion artefacts with improved accuracy.

SUMMARY OF THE INVENTION

The invention is defined by the claims.

According to examples in accordance with an aspect of the invention, there is provided a physiological sensor apparatus for measuring at least one physiological parameter, adapted for controlling the positioning of a sensing surface against a human tissue receiving surface, comprising:

said sensing surface;
an electroactive polymer structure adapted to deform in response to the application of an electrical signal, to thereby manipulate a positioning of the sensing surface; and
a controller, adapted to:

provide (or apply) a signal, such as an electrical signal, composed of a superposed actuation signal and AC sensing signal to the electroactive polymer structure, the actuation signal for stimulating a deformation of the structure to thereby manipulate the sensing surface to apply an actuation force to the receiving surface, and the AC sensing signal for facilitating pressure sensing and having an AC frequency harmonic with either a resonance or anti-resonance frequency of the electroactive polymer structure;
monitor an impedance exhibited by the electroactive polymer structure over time, to thereby provide an indication of a returning force exerted on the electroactive polymer structure by the receiving surface over time, and
adjust a magnitude of the applied actuation signal in dependence upon the measured impedance and/or the returning force to thereby adjust the positioning of the sensing surface against the receiving surface.

The invention is based on the use of electroactive polymer (EAP) structures to achieve simultaneous actuation and sensing. This enables real-time feedback to be obtained on the extent of actuation of the structure, which may then be used to adjust the actuation signal supplied to the structure. Since actuation and sensing is simultaneous and spatially overlapping, more accurate control over pressure application and position adjustment is achieved.

Electroactive polymers (EAPs) are an emerging class of materials within the field of electrically responsive materials. EAPs can work as sensors or actuators and can easily be manufactured into various shapes allowing easy integration into a large variety of systems. Advantages of EAPs include low power, small form factor, flexibility, noiseless operation, accuracy, the possibility of high resolution, fast response times, and cyclic actuation.

Application of a small force to certain classes of EAP generates an electrical signal in response, which allows a single EAP structure to be used both for actuation and for sensing. However, state of the art EAP based actuator/sensors have typically provided sensing and actuation functions which are separated from one another, either physically - wherein a different region or portion of the device is used for sensing as for actuation, with separately provided electrical connection to each for example - or temporally, wherein the single device is sequentially alternated between a sensing and an actuation function.

By superposing a low-amplitude, high frequency sensing signal on top of a higher amplitude primary actuation signal, sensing and actuation functions may be achieved simultaneously, The amplitude of the sensing signal may be significantly less than that of the actuation signal, for example <10% of that of the actuation signal, for example <1% of that of the actuation signal. In this way the deformation response in the electroactive polymer (EAP) structure may be negligible for the sensing signal compared to that stimulated by the actuation signal. Hence precision, accuracy and stability of the device as an actuator is not compromised.

The actuation signal is for example a DC signal (although with DC level which may vary in dependence on the actuation desired). The actuation signal could however be AC signal, but with an AC frequency much lower than that of the sensing signal.

When the sensing is applied at a frequency matching the mechanical resonance or anti-resonance frequency of the EAP structure (or one of their harmonics), a mechanical standing wave is established in the material which in turn affects the electrical characteristics of the material. In particular, the impedance of the material is lower for a drive signal matching the resonance frequency, due to the mechanical vibration being in-phase with the electrical driving signal. Conversely, the impedance of the material is higher for a drive signal matching the anti-resonance frequency of the material, due to the mechanical vibration being out of phase with the electrical driving signal. In the context of the present application the term AC frequency "harmonic" includes a series of positive integer multiples of the fundamental frequency, including the fundamental frequency (resonance or anti-resonance frequency of the electroactive polymer structure). Therefore, the AC sensing signal for facilitating pressure sensing has the AC frequency belonging to a frequency range defined by harmonic of either the resonance or anti-resonance frequency: the series of positive integer multiples of the fundamental frequency (including the fundamental frequency) being either the resonance or anti-resonance frequency.

Any pressure or other mechanical load applied to the device may cause a damping in the material, causing its resonance or anti-resonance frequency (and the harmonics) to shift away from their ordinary un-damped values, thereby inducing a disparity between the high-frequency driving signal and the frequency of mechanical vibration. This disparity can be detected in changes in the exhibited impedance values across the EAP structure.

In the case that the driving signal is applied at a frequency matching the (undamped) anti-resonance frequency, for example, the sudden mismatch induced by the applied load may then be detected as a consequent drop in impedance as measured across the EAP structure. Alternatively, in the case that the driving signal is applied matching the (undamped) resonance frequency, the mismatch may be detected as a consequent jump in impedance measured across the EAP structure. In either case, the high frequency signal, in this way, allows for sensing of external pressure and load applied to the device at the same time as actuation.

In this way, monitoring of the exhibited impedance allows an indication of a returning force exerted on the EAP structure to be obtained. More particularly, it may allow an indication of the magnitude of the applied force to be obtained. This may be a direct or indirect indication for instance. Monitoring of the impedance may in this way provide a measure of the exerted returning force, where measure is to be interpreted broadly as implying a (direct or indirect) indication of a level or magnitude or extent of the force exerted. The indication of the force may be a quantitative indication or measure in this sense. A quantitative measure or indication may be a numerical measure for example, or may provide more indirect information from which a numerical indication of the force might be extractable.

The impedance of the EAP structure may be monitored in example embodiments by monitoring the voltage and current of the sensing signal over time. Alternatively, impedance may be monitored by monitoring the voltage and current of the high magnitude actuation signal. Alternatively still, impedance may be monitored by means a separate monitoring circuit, having separate dedicated measurement electrodes in connection with the structure and an analysis circuit for determining impedance.

A numerical measure of the applied force, pressure or load may be obtained in examples by means of some pre-calibration process, whereby forces across a range of magnitudes are applied to the actuator, at a range of different actuation voltages, and the corresponding exhibited impedance values recorded. These impedance values may then be used as a reference during operation to provide a means of relating measured impedance values at a given actuation voltage to an applied force or load.

Measuring a returning force simultaneously with actuation allows instantaneous feedback to be acquired on the state of actuation, or upon the interaction between the EAP structure and the receiving surface. Far more precise control over the actuator positioning and movement is therefore achievable, enabling adjustments of the positioning of the sensor surface against the skin (for example to correct for motion artefacts) to be performed with far greater precision. Simultaneous actuation and sensing also enables the single EAP structure to provide load sensing and actuation at the same location, hence providing improved accuracy especially in the case of tonometry applications.

The controller is adapted to monitor the exhibited impedance values over time, and to adjust the applied actuation voltage (and hence the force applied to the receiving surface by the EAP structure) in dependence upon these values, or upon the magnitude of the returning force indicated by the values. In this way, embodiments of the invention implement an integrated feedback system, whereby the induced actuation level of the EAP structure is automatically adjusted in dependence upon the sensed contact force between the sensing surface by the human tissue receiving surface.

The controller may be adapted to detect changes in the contact force exerted by the receiving surface, and to adjust the actuation signal in dependence upon these.

Such changes may typically indicate changes in the positioning of the sensing surface relative to the receiving surface, which might cause motion artefacts. The integrated feedback system provided by the invention may achieve automated compensation for such movement, by automatically adjusting the actuation signal in response.

In accordance with at least one set of embodiments, the controller may be adapted to adjust the magnitude of the actuation signal so as to maintain a steady actuation force applied against the receiving surface and/or so as to maintain a steady returning force, or component thereof, applied against the sensing surface.

This may provide compensation for motion artefacts, since movement of the sensing surface relative to the receiving surface may typically result in a change in the measured return force exerted by the receiving surface. Adjustment to maintain a constant return force may ensure maintenance of a uniform positioning.

A steady force may imply for instance a force which is constant, substantially constant, constant within certain tolerances or parameters, or uniform or substantially uniform.

As stated above, a force may be derived from the impedance values by means of a pre-calibration process. A physical model, or a standard set of reference values, might also be used to derive values of applied force from the measured impedance values.

In alternative examples, the actuation voltage may be adjusted in dependence directly upon the measured impedance values, the impedance values providing a proxy measure of an applied force.

In accordance with a further set of embodiments, the controller may be adapted to adjust the magnitude of the actuation signal so as to maintain a steady relative distance between the sensing surface and the receiving surface and/or a point or body beneath the receiving surface.

The point or body beneath the receiving surface may be a vein, artery, bone, muscle or some other anatomical feature of structure for instance. This may be advantageous in cases where blood pressure or pulse rate are parameters of interest.

The controller may be adapted to decrease the magnitude of the actuation signal in response to falling or rising impedance values, and/or to increase the magnitude of the control signal in response to rising or falling impedance values.

In the case that the AC sensing signal has a frequency matching the anti-resonance frequency of the EAP structure the actuation signal may be decreased in response to falling impedance values, and/or increased in response to rising impedance values. For an AC signal applied at anti-resonance, impedance decreases when external forces acting on the structure increase. Decreasing impedance values may therefore indicate that the sensing surface is being applied to the receiving surface with greater force (for example as a result of accidental movement by the user). By decreasing actuation level in response, this change may be compensated for, by applying the sensing surface to the receiving surface with reduced force.

For an AC signal at resonance, the impedance responses are reversed, and hence in this case rising impedance values may indicate that the sensing surface is being applied to the receiving surface with greater force, and that therefore the actuation voltage should be decreased in response.

According to a further set of examples, the controller may be adapted to decrease the magnitude of the actuation signal in response to impedance values falling below or rising above a defined threshold, and/or to increase the magnitude of the actuation signal in response to impedance values rising above or falling below a defined threshold.

In any embodiment, the measured returning force exerted by the receiving surface may in general be composed of multiple components. It may firstly comprise a reaction force component, representing a force exerted by the receiving surface in reaction to the applied actuation force, arising as a result of Newton's third law. This is typically expected to have a magnitude approximately equal and opposite to the applied force.

There may secondly be a physiological component, representing a force or pattern of forces caused by some physiological action, interaction or phenomenon. In particular, a measured returning force may for example include a fluctuating or oscillating force component caused by a blood pressure or by the pulsing of blood through a vein or artery beneath the receiving surface. This is particularly the case in tonometry applications in which a force is applied directly onto an artery or vein, and where the returned force applied by the receiving surface will typically include a component caused by blood flow through the blood vessel below.

In many cases, it may be desirable to separate these two components, so as thereby to provide a measure of the physiological parameter in isolation from the base-line reaction force. Accordingly, in accordance with at least a subset of embodiments, the sensor apparatus may further comprise a filter circuit adapted to filter the obtained impedance values so as to extract a reaction force component, representing forces exerted by an intrinsic elasticity of the receiving surface, and/or a physiological component, representing forces exerted as a result of one or more physiological phenomena.

The filter circuit in particular, may include a low-pass filter to extract the reaction force component, and/or a high-pass filter to extract the physiological component. The exhibited reaction force will typically be roughly static over time, and hence have a low or close to zero frequency. A low-pass filter would hence enable extraction of this component. The physiological component may, in at least some cases, be oscillatory or time-varying in nature. This is especially the case for blood pressure or pulse rate. Hence a high-pass filter would enable extraction of this component. The filter circuit may include both a high-pass and low-pass filter (connected in parallel) to enable both components to be extracted.

The physiological component may in examples represent forces associated with blood pressure and/or a cardiovascular pulse wave.

The controller may in accordance with examples adapted to adjust the magnitude of the actuation signal in dependence upon either the reaction force component or the physiological component of the measured impedance values.

As noted above, the reaction force component may typically be expected to be equal and opposite in magnitude to the applied actuation force. Hence, the reaction force component may provide a proxy measure of the applied actuation force itself. This hence provides a means for obtaining direct feedback regarding the extent of actuation of the EAP structure and its positioning relative to the receiving surface.

The reaction force component of the impedance values may comprise a particular set of impedance values, excluding certain others. The controller furthermore may be adapted to adjust the magnitude of the actuation signal in dependence upon this extracted set of impedance values alone. Reference above to 'rising impedance values' or 'falling impedance values' is therefore to be understood in this context as potentially including cases where only an extracted set of impedance values in considered.

The EAP structure itself may in some cases be configured to acquire measurements of one or more physiological parameters. It may for example be applied to measure blood pressure and related parameters in accordance with a tonometric method. Since the physiological and reaction force components may be separated in some embodiments, the EAP structure may simultaneously provide actuation feedback (by means of the reaction force component) and physiological parameter measurement, for example of pulse rate or blood pressure (by means of the physiological component). This can be achieved at the same time, and in the same location on the receiving surface, as actuation.

Hence, in accordance with at least one set of examples, the sensing surface may be a surface of the EAP structure itself, wherein the structure is configured to directly acquire measurements for determining physiological parameters.

In an alternative set of examples, the sensing surface may be a surface of a further auxiliary sensing component, arranged in mechanical co-operation with the electroactive polymer structure, and adapted to measure one or more physiological parameters.

This may for example be an optical-based device, such as a PPG device. It may in further examples be any device or module for measuring or sensing one or more physiological parameters, such as an EEG (electroencephalography), ECG (electrocardiogram) and/or $S_PO_2$ (peripheral oxygen saturation) sensor. The EAP structure may be arranged so as to be operable to manipulate positioning of this further sensor relative to the receiving surface and/or change a force or pressure with which it is applied to the receiving surface.

In accordance with at least one set of embodiments, the controller may be adapted to provide or apply a actuation signal which steadily decreases in magnitude over a defined time period, and is further adapted to process the measured impedance values over said time period to detect and measure oscillatory changes in the value over time, these changes being indicative of oscillating blood-vessel walls caused by blood pressure.

The sensor apparatus may in examples comprise an array of electroactive polymer structures, each independently controllable by the controller to manipulate a respective sensing surface to apply a force at a respective point on the receiving surface and to measure a returning force exerted by the receiving surface at said point.

According to one or more embodiments, the electroactive polymer structure/s and/or the controller may be mounted to a flexible carrier for application to a region of the receiving surface. A flexible carrier may enable the apparatus to be soundly pressed, applied or adhered to the receiving surface. It may form part of a wearable device for example, wherein the sensor apparatus can be secured or fixed against a portion of a subject's skin. A wearable device may further prevent unintended movement of the device during operation, or may assist in providing a firm supporting (backing) surface against which the actuation forces may be exerted.

In one or more embodiments, the sensor apparatus may further comprise a layer of piezoelectric material mechanically adhered to the electroactive polymer structure and/or to the receiving surface, and electrically coupled with the controller, the layer being operable to measure an applied force exerted upon it by the receiving surface. This may provide an additional means for measuring forces exerted by the receiving surface. This may improve sensitivity or provide further information which may assist in measuring a physiological parameter or in measuring a reaction force applied by the receiving surface.

Other sensing elements may additionally or alternatively be incorporated, such as a strain gauge, or small solid pressure sensors such as pressure cells or integrated SMD components.

The electroactive polymer structure may in examples comprise a relaxor ferroelectric polymer. These are non-ferroelectric in the absence of an applied DC voltage, and hence exhibit in this state no electromechanical coupling. When a DC voltage is applied, electromechanical coupling becomes non-zero, and can be measured by applying (or providing) a small-amplitude high frequency signal on top of the DC bias. Relaxor ferroelectric materials are hence ideally suited for embodiments of the present invention.

Examples in accordance with a further aspect of the invention provide a method of adjusting a physiological sensing apparatus to optimise a positioning of a sensing surface against a human tissue receiving surface, the apparatus comprising:

said sensing surface;
an electroactive polymer structure adapted to deform in response to the application of an electrical signal,
the method comprising:

providing (or applying) a signal, such as an electrical signal, composed of a superposed actuation signal and AC sensing signal to the electroactive polymer structure, the actuation signal for stimulating a deformation of the structure to thereby manipulate the sensing surface to apply an actuation force to the receiving surface, and the AC sensing signal for facilitating pressure sensing and having an AC frequency harmonic with either a resonance or anti-resonance frequency of the electroactive polymer structure;
monitoring an impedance exhibited by the electroactive polymer structure over time, to thereby provide an indication of a returning force exerted on the electroactive polymer structure by the receiving surface over time, and
adjusting a magnitude of the applied actuation signal in dependence upon the measured impedance to thereby adjust the positioning of the sensing surface against the receiving surface.

Examples in accordance with a further aspect of the invention also provide a computer program comprising computer program code means which is adapted, when said program is run on a computer, to implement the method of adjusting a physiological sensing apparatus set out above.

These and other aspects of the invention are apparent from and will be elucidated with reference to the embodiments described hereinafter.

It will be appreciated by those skilled in the art that two or more of the above-mentioned options, implementations, and/or aspects of the invention may be combined in any way deemed useful.

BRIEF DESCRIPTION OF THE DRAWINGS

These and other aspects of the applicator device, the system and the method according to the invention will be further elucidated and described with reference to the accompanying drawings, in which:

Figure 1 illustrates the general principles of tonometry-based blood pressure measurement;
Figure 2 shows a known electroactive polymer device which is not clamped;
Figure 3 shows a known electroactive polymer device which is constrained by a backing layer;
Figure 4 illustrates a simple capacitor-resistor circuit;
Figure 5 illustrates a simple first example of a sensing apparatus in accordance with one or more embodiments of the invention;
Figure 6 shows changes in resistance and capacitance with frequency for an example EAP structure;
Figure 7 shows a graph illustrating series resistance (of an example EAP structure) versus sensor signal frequency for two different fixed actuation voltages;
Figure 8 shows a graph illustrating the difference between the two signal traces of Figure 7;
Figure 9 shows a graph illustrating the effect of an applied load force on the measured resistance values across a range of sensor signal frequencies;
Figure 10 shows a graph illustrating measured resistance values over time (of an example EAP actuator), wherein a load is applied at two distinct points in time;
Figure 11 shows a second example sensor apparatus, comprising a further sensing element;
Figure 12 shows application of a third example sensor apparatus to perform tonometry-based blood pressure measurement;
Figure 13 shows a fourth example sensor apparatus, comprising an array of EAP structures mounted to a flexible carrier; and
Figure 14 shows an EAP structure of a fifth example sensor apparatus, comprising a secondary piezoelectric sensing layer.

DETAILED DESCRIPTION OF THE EMBODIMENTS

The invention provides a physiological sensor apparatus offering auto-adjustment of a physiological sensing surface relative to a human tissue receiving surface. The apparatus includes an electroactive polymer (EAP) structure, operable to perform actuation and pressure sensing simultaneously, via application of an electrical signal composed of superposed actuation and AC sensing signals. Actuation enables controlled adjustment of the positioning of a sensing surface against the tissue receiving surface. Sensing provides a simultaneous real-time measure of the magnitude of a returning force applied to the sensing surface by the receiving surface. This returning force provides feedback on the state of positioning of the sensing surface. A controller is adapted to adjust the actuation signal in dependence upon the sensing data, to thereby adjust the positioning of the sensing surface in real-time.

In embodiments, the returning force may in addition provide a direct or indirect measure of a physiological parameter. This may be extracted by means of a filter circuit in examples.

The invention is based on the use of electroactive polymers to provide simultaneous integrated actuation and pressure/load sensing. Actuation and sensing in the same place and at the same time improves accuracy of actuation, by enabling real-time feedback on an extent of actuation, and improves accuracy in measurement of any physiological parameter, since measurement can be performed in exactly the same location as a stimulating pressure is being applied.

This is particularly pertinent in the case of tonometry methods (as discussed above).

Electroactive polymers (EAPs) are an emerging class of materials within the field of electrically responsive materials. EAPs can work as sensors or actuators and can easily be manufactured into various shapes allowing easy integration into a large variety of systems.

Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation.

The improved performance and particular advantages of EAP material give rise to applicability to new applications.

An EAP device can be used in any application in which a small amount of movement of a component or feature is desired, based on electric actuation. Similarly, the technology can be used for sensing small movements.

The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 1MHz, most typically below 20 kHz.

Devices using electroactive polymers can be subdivided into field-driven and ionic-driven materials.

Examples of field-driven EAPs include Piezoelectric polymers, Electrostrictive polymers (such as PVDF based relaxor polymers) and Dielectric Elastomers. Other examples include Electrostrictive Graft polymers, Electrostrictive paper, Electrets, Electroviscoelastic Elastomers and Liquid Crystal Elastomers.

Examples of ionic-driven EAPs are conjugated/conducting polymers, Ionic Polymer Metal Composites (IPMC) and carbon nanotubes (CNTs). Other examples include ionic polymer gels.

Field-driven EAPs are actuated by an electric field through direct electromechanical coupling. They usually require high fields (volts per meter) but low currents. Polymer layers are usually thin to keep the driving voltage as low as possible.

Ionic EAPs are activated by an electrically induced transport of ions and/or solvent. They usually require low voltages but high currents. They require a liquid/gel electrolyte medium (although some material systems can also operate using solid electrolytes). Both classes of EAP have multiple family members, each having their own advantages and disadvantages.

A first notable subclass of field driven EAPs are Piezoelectric and Electrostrictive polymers. While the electromechanical performance of traditional piezoelectric polymers is limited, a breakthrough in improving this performance has led to PVDF relaxor polymers, which show spontaneous electric polarization (field driven alignment). These materials can be pre-strained for improved performance in the strained direction (pre-strain leads to better molecular alignment). Normally, metal electrodes are used since strains usually are in the moderate regime (1-5%). Other types of electrodes (such as conducting polymers, carbon black based oils, gels or elastomers, etc.) can also be used. The electrodes can be continuous, or segmented.

Another subclass of interest of field driven EAPs is that of Dielectric Elastomers. A thin film of this material may be sandwiched between compliant electrodes, forming a parallel plate capacitor. In the case of dielectric elastomers, the Maxwell stress induced by the applied electric field results in a stress on the film, causing it to contract in thickness and expand in area. Strain performance is typically enlarged by pre-straining the elastomer (requiring a frame to hold the pre-strain). Strains can be considerable (10-300%). This also constrains the type of electrodes that can be used: for low and moderate strains, metal electrodes and conducting polymer electrodes can be considered, for the high-strain regime, carbon black based oils, gels or elastomers are typically used. The electrodes can be continuous, or segmented.

A first notable subclass of ionic EAPs is Ionic Polymer Metal Composites (IPMCs). IPMCs consist of a solvent swollen ion-exchange polymer membrane laminated between two thin metal or carbon based electrodes and requires the use of an electrolyte. Typical electrode materials are Pt, Gd, CNTs, CPs, Pd. Typical electrolytes are Li+ and Na+ water-based solutions. When a field is applied, cations typically travel to the cathode side together with water. This leads to reorganization of hydrophilic clusters and to polymer expansion. Strain in the cathode area leads to stress in rest of the polymer matrix resulting in bending towards the anode. Reversing the applied voltage inverts bending. Well known polymer membranes are Nafion® and Flemion®.

Another notable subclass of Ionic polymers is conjugated/conducting polymers. A conjugated polymer actuator typically consists of an electrolyte sandwiched by two layers of the conjugated polymer. The electrolyte is used to change oxidation state. When a potential is applied to the polymer through the electrolyte, electrons are added to or removed from the polymer, driving oxidation and reduction. Reduction results in contraction, oxidation in expansion.

In some cases, thin film electrodes are added when the polymer itself lacks sufficient conductivity (dimension-wise). The electrolyte can be a liquid, a gel or a solid material (i.e. complex of high molecular weight polymers and metal salts). Most common conjugated polymers are polypyrolle (PPy), Polyaniline (PANi) and polythiophene (PTh).

An actuator may also be formed of carbon nanotubes (CNTs), suspended in an electrolyte. The electrolyte forms a double layer with the nanotubes, allowing injection of charges. This double-layer charge injection is considered as the primary mechanism in CNT actuators. The CNT acts as an electrode capacitor with charge injected into the CNT, which is then balanced by an electrical double-layer formed by movement of electrolytes to the CNT surface. Changing the charge on the carbon atoms results in changes of C-C bond length. As a result, expansion and contraction of single CNT can be observed.

Figures 2 and 3 show two possible operating modes for an EAP device.

The device comprises an electroactive polymer layer 26 sandwiched between electrodes 22, 24 on opposite sides of the electroactive polymer layer 26.

Figure 2 shows a device which is not clamped. A voltage is used to cause the electroactive polymer layer to expand in all directions as shown.

Figure 3 shows a device which is designed so that the expansion arises only in one direction. The device is supported by a carrier layer 28. A voltage is used to cause the electroactive polymer layer to curve or bow.

Together, the electrodes, electroactive polymer layer, and carrier may be considered to constitute the overall electroactive polymer structure.

The nature of this movement for example arises from the interaction between the active layer which expands when actuated, and the passive carrier layer. To obtain the asymmetric curving around an axis as shown, molecular orientation (film stretching) may for example be applied, forcing the movement in one direction.

The expansion in one direction may result from the asymmetry in the EAP polymer, or it may result from asymmetry in the properties of the carrier layer, or a combination of both.

An electroactive polymer structure as described above may be used both for actuation and for sensing. The most prominent sensing mechanisms are based on force measurements and strain detection. Dielectric elastomers, for example, can be easily stretched by an external force. By putting a low voltage on the sensor, the strain can be measured as a function of voltage (the voltage is a function of the area).

Another way of sensing with field driven systems is measuring the capacitance-change directly or measuring changes in electrode resistance as a function of strain.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured. It has also been shown that the resistance of CNTs change when in contact with gaseous molecules (e.g. $O_2$, $NO_2$), making CNTs usable as gas detectors.

The present invention makes use in particular of a different sensing mechanism, whereby an EAP is operable to achieve simultaneous actuation and pressure/load sensing based on application of a superposed high magnitude actuation signal and low-amplitude AC sensing signal. The principles by which simultaneous sensing and actuation are achieved in embodiments of the invention will now be described in detail.

As is well known, application of a DC bias (or slowly varying AC bias) stimulates deformation of an EAP, the extent of deformation varying in dependence upon the magnitude of the applied electrical signal. Superposing a high frequency AC signal atop the bias also stimulates a mechanical deformation response in the material, but a deformation response which is periodic, rather than fixed (i.e. an oscillation). The maximal amplitude of the high frequency signal should be maintained at a level significantly lower than the magnitude of the bias signal (for example two orders of magnitude lower than that of the bias signal, for example, 1% of that of the bias signal). As a result, the corresponding displacement amplitude of the stimulated deformation is effectively negligible compared to the primary actuation displacement. The accuracy and stability of the actuation is therefore not affected by the superposition of the sensing signal.

The overlay of a low-amplitude oscillation signal on top of the bias allows for an electrical feedback mechanism to be incorporated within the primary actuator driving mechanism itself. At certain frequencies, in particular at frequencies which match or are harmonic with the mechanical resonance frequency of the EAP structure, a small mechanical standing wave is established in the material of the actuator. This in turn influences the electrical characteristics of the material. When the sensing signal is driven at the resonance frequency of the material, the corresponding impedance of the material is lower (compared to when driven at non-resonance) due to the mechanical vibration being in-phase with the electrical driving signal.

The mechanical resonance frequency of a structure is the frequency at which a structure will naturally tend to oscillate, upon being displaced from its equilibrium position, and is determined by intrinsic structural properties of the structure (e.g. geometry, size, shape, thickness etc.). The mechanical oscillation of the EAP structure will not necessarily follow the drive frequency of the electrical signal applied to it, but will tend to fall back to its natural resonance frequency, with the drive frequency interfering with that oscillation either constructively or destructively, depending upon whether the driving frequency is either in phase or out of phase with this natural frequency.

When the high-frequency signal is driven at the anti-resonance frequency of the EAP structure, the impedance of the EAP is higher, due to the mechanical vibration of the material being out of phase with the oscillation of the drive signal (the electrically induced mechanical strains are out of phase with the electrical excitation). In other words, whenever, for instance, a positive current is being applied to the EAP by the drive signal, the out of phase mechanical strains are at the same moment inducing a current in the opposite direction (i.e. out of phase behaviour). In the ideal (model) case these opposing currents cancel each other out, and no current can flow at all (i.e. infinite impedance), but in real-world scenarios no full cancellation occurs and this effect is measured as an (effective) higher resistance of the electrical current (i.e. higher impedance). In particular, when the signal is driven at the anti-resonance frequency of the actuator material, the impedance of the EAP is at a maximum.

The relationship may be further understood by considering equation (3) below. The impedance of an ideal EAP at resonance and anti-resonance depends on the particular type or mode of deformation. It is most common to bring the EAP into lateral resonance (i.e. length or width). The impedance of the EAP is governed by the dielectric properties of the material and the electromechanical coupling and electrical and mechanical losses. For simplicity, when ignoring the electrical and mechanical losses, for an EAP with length $l$, width $w$ and thickness $t$, deforming in lateral extension, the impedance of the EAP is given by:

$$Z(\omega) = \cfrac{1}{i\omega\frac{lw}{t}\varepsilon_{33}^T\left[(k_{31})^2\cfrac{\tan\left(\frac{\omega l}{2}\left(\rho s_{11}^E\right)^{1/2}\right)}{\frac{\omega l}{2}\left(\rho s_{11}^E\right)^{1/2}\gamma\alpha^{(E)}}+1-(k_{31})^2\right]} \qquad (3)$$

where $\varepsilon^T{}_{33}$ is the dielectric constant, $k_{31}$ is the lateral electromechanical coupling factor, $p$ is the density of the EAP and $s^E{}_{11}$ is the compliance in the lateral direction. At anti-resonance frequency, $\omega_a$, $\tan\left(\frac{\omega l}{2}\left(\rho s_{11}^E\right)^{1/2}\right) = 0$ and $Z$ is highest.

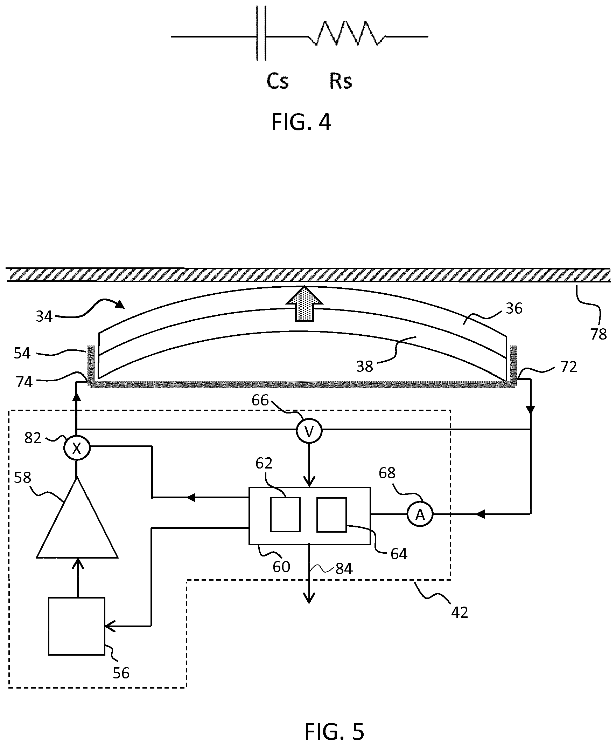

A real EAP has losses and can be modelled or represented by a capacitor with a resistor in series, the resistance of which is greatest at the anti-resonance frequency. This is illustrated in Figure 4. In the descriptions which follow, therefore, 'impedance' and 'series resistance' ($R_S$) may be used interchangeably with reference to the device. However, series resistance is to be understood in this context as referring simply to a model in which the actuator/sensor is represented electronically by a capacitor in series with a resistor, having resistance $R_S$.

In consequence of the above-described relationship between impedance and resonance, when the drive signal is being driven at the anti-resonance frequency, any small deviations which occur in its frequency away from anti-resonance will be detectable in a corresponding sharp drop-off in the measurable impedance of the EAP structure. It is this physical effect which allows mechanical sensing to be achieved. Application of pressure or load to the EAP structure results in a dampening of any resonance effects occurring within the material. If the drive signal is oscillating at the anti-resonance or resonance frequency of the material when the load is applied, the dampening effect will be identifiable within real-time measurements of the EAP impedance (i.e. series resistance $R_S$), as the sudden cessation of resonance will effect a consequent sharp decline in the impedance. Hence by monitoring the impedance of the structure over time, while the actuator is in operation, pressures and loads applied to the structure can be sensed, and in some cases quantitatively measured (as will be described below).

In particular, the impedance of the EAP structure may be monitored in example embodiments by monitoring the voltage and current of the high-frequency sensing signal over time. Alternatively, impedance may be monitored by monitoring the voltage and current of the high magnitude actuation signal. Alternatively still, impedance may be monitored by means a separate monitoring circuit, having separate dedicated measurement electrodes and analysis circuit.

The frequency of the high-frequency sensing signal may typically be in the range of 1kHz to 1MHz, depending on the particular geometry of the actuator. Note that in the case that the actuator drive signal comprises an AC drive signal, the frequency of this signal is significantly lower than that of the alternating sensing signal. The (low frequency) actuation voltage in this case may for example be at least two orders of magnitude lower than the high frequency signal voltage, to avoid interference of the actuator signal with the measurement signal.

Figure 5 schematically illustrates a first example configuration for an EAP structure as implemented in embodiments of the invention. An EAP structure 34, comprising an EAP material layer 36 disposed atop a lower passive carrier layer 38 is held within a housing 54, and electrically coupled with a controller 42. The controller in the example of Figure 5 comprises both signal generation elements and signal processing and analysis elements.

An actuator control element 56 generates a high-amplitude actuator drive signal (for example a fixed DC bias voltage) which is transmitted to a signal amplifier device 58. A sensor control element 60 comprises both a driver element 62 for generating the high frequency sensing signals, and a processing element 64 for analysing electrical properties of the sensing signal after passage across the EAP structure. The sensor control element 60 (and in particular, the processing element 64 of sensor control element) is signally coupled with the actuator control element 56 for communicating the signal analysis information to the actuator control element, for use by actuator control element in controlling the magnitude of the actuator drive signal.

To facilitate analysis of the electrical properties of the sensing signal, the controller 42 further comprises a voltmeter 66, connected across the EAP structure 34, and an ammeter 68 connected in series between the outgoing electrical terminal 72 of the actuator and the sensor control element 60. The voltmeter 66 and ammeter 68 are both signally connected with the sensor control element 60, such that data generated by them may be utilised by the processing element 64 in order to determine an impedance of the EAP structure 34 (that is, the equivalent series resistance $R_S$ where the device is modelled as an ideal capacitor with a resistor in series, i.e. the real part of the complex impedance).

Drive signals generated by the actuator control element 56 (once amplified by amplifier 58) and sensor control element 60 are superposed by a combiner element 82. The combiner element may for example comprise a DC-bias block in the case that the actuator drive signal is a DC signal. The combiner element may in further examples simply comprise a series junction between the amplifier 58 and the sensor control element 60.

The sensor control element 60 may be adapted to locally amplify the generated sensing signal, in advance of outputting it to the combiner element.

The combined drive signal is then transmitted to ingoing terminal 74 of the EAP structure 34. The EAP structure may comprise electrodes across a top and bottom planar surface for generation of an electric field across the EAP layer. The high magnitude DC component of the combined drive signal stimulates a deformation response in the EAP structure, as illustrated in Figure 5. The EAP structure is held within shown housing 54. For the most reproducible (i.e. reliable/accurate) results, the EAP structure may be clamped in position. For example, the EAP structure may be clamped within housing 54, and the housing then positioned so as to align the device with a target actuation and sensing area of a human tissue receiving surface 78.

For illustration, a target area of a receiving surface 78 is shown in Figure 5, wherein the EAP structure 34 is deformed by the DC (or slowing varying AC) drive signal to apply pressure to the target area. In examples, the target area may comprise a region of a subject's skin for instance. The EAP structure is shown applying pressure directly to a region of a user's skin. However, this is by way of illustration of the actuation and sensing principles only, and in further examples (to be described in greater detail below), the EAP structure may alternatively apply pressure to a secondary sensing surface of a further sensing element, this element being pressed against the receiving surface.

A returning force exerted by the skin upon the EAP structure (or further sensing surface in the case that the arrangement includes a further sensing element) may be measured simultaneously with actuation.

The low-amplitude AC component of the drive signal stimulates a low amplitude periodic response in the EAP layer 36, for example oscillating the structure at its resonance or anti-resonance frequency.

The voltage of the combined drive signal and the resulting current are fed to sensor control element 60. Typically the AC currents may be in the range of 0.1mA to 1mA, but may be up to 10mA. Higher currents may cause overheating.

The processing element 64 of sensor control element 60 may use measurements provided by voltmeter 66 and ammeter 68 in order to determine an impedance across the actuator, as experienced by the applied drive signal(s), for example a complex impedance In simple examples, a series resistance alone may be determined, or a series resistance may be extracted from a determined complex impedance. In further examples, a reactance may also be determined, for example by means of extraction from a determined complex impedance, Reactance may be of interest in many cases, in particular where the EAP structure comprises particularly thin layers of EAP material, and is therefore liable to exhibit relatively high reactance values.

For the purposes of simplicity and clarity, it will be assumed for the present example that only a series resistance is determined. However, the explanations that follow are to be understood as applicable without loss of generality to cases where an impedance is determined.

A series resistance may be determined in real time, and monitored for example for sudden changes in resistance, which, as explained above, may be used to indicate the presence and magnitude of loads and pressures applied to the EAP structure 34. The sensing element may be adapted to derive from the determined series resistance values numerical values for the corresponding applied force or pressure indicated by the series resistance. These values, or in alternative examples, the resistance values alone, are output via a signal output 84.

In accordance with certain examples, the sensor control element 60 and/or the actuator control element 56 may be further provided with a signal input for communicating user control signals. User control signals may be for adjusting a mode or operational setting of the sensor apparatus for instance.

The sensor control element 60 (and in particular, the processing element 64 of sensor control element) is further signally coupled with the actuator control element 56, and the determined series resistance or calculated force values communicated to the actuator control element. The actuator control element is adapted to control the magnitude of the actuator drive signal in dependence upon the resistance or force values. In this way application of a sensing surface of the EAP structure, or an additional auxiliary sensing device, is adjusted and controlled in dependence upon force feedback data provided through analysis of the series resistance of the applied signal(s).

In some cases, the sensor control element 60 may further comprise one or more filter circuits for extracting different components of the obtained resistance or force measures. For example, there may be included a low-pass filter to extract a component corresponding to an elastic reaction force of the skin surface 78 to application of an actuating force. There may be included a low-pass filter to extract a component corresponding to one or more physiological parameters such as blood pressure or pulse rate. In this case, apparatus may be applied to the subject's skin at an appropriate position above an artery or vein for instance. This will be described in greater detail below.

The controller may in examples comprise or consist of a microprocessor. The various components of the controller illustrated in Figure 5 may in this case be understood as representing merely notionally separate parts of the functioning of such a microprocessor. The physical structure may vary, while incorporating the functional structure illustrated by Figure 5.

As discussed above, the sensor driver element 62 is adapted to generate an AC signal for application to the EAP structure 34 having a frequency which is resonant with the resonance or anti-resonance frequency of the EAP structure. At the anti-resonance frequency, the impedance of the EAP structure is at a minimum. At the resonance frequency, the impedance of the EAP structure is at a maximum. Equivalently, the EAP can be modelled or represented by a capacitor with a resistor in series (as shown in Figure 4), the resistance of which is greatest at the anti-resonance frequency, and lowest at the resonance frequency.

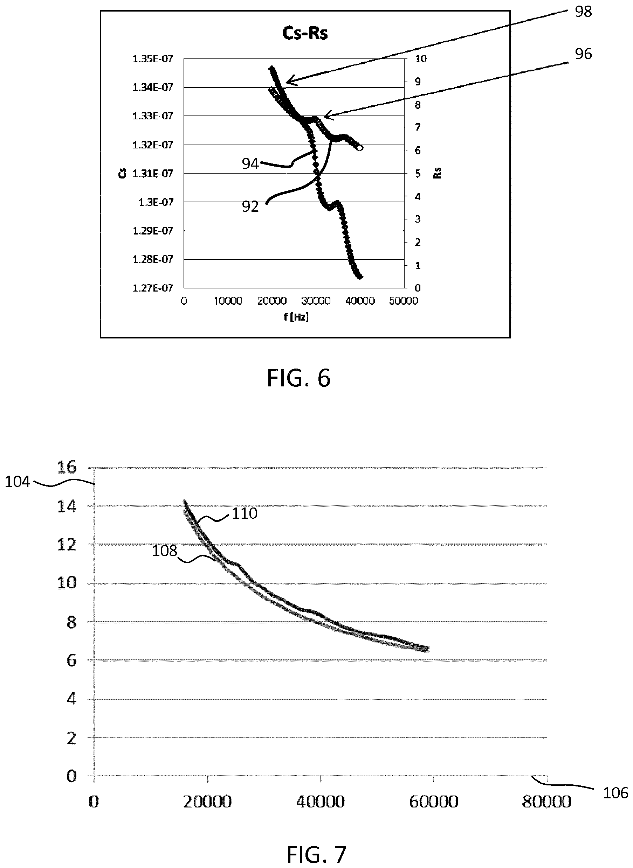

This is illustrated in Figure 6, for anti-resonance in particular. The graph shows measured values of both series resistance and capacitance for a sample EAP across a continuous sweep of different applied AC signal frequencies. Measured series resistance (in Ohms) is shown on one y-axis, the measured capacitance (in Farads) is shown on another y-axis and the sensor signal frequency (in Hz) on the x-axis.

Plot 92 is the resistance and plot 94 is the capacitance. Arrow 96 indicates a strong local peak in the measured resistance, occurring at a frequency of approximately 29.8 kHz. An arbitrary off-(anti)resonance point, occurring at a frequency of approximately 20 kHz is indicated by arrow 98, by way of comparison. The peak 96 represents the anti-resonance peak for the particular EAP sampled, for which resistance is at a local maximum. The anti-resonance frequency is therefore 29.8 kHz for this sampled EAP. The plots are for a bias voltage of 200V.

In order to initially configure the EAP structure 36 in a state of resonance or anti-resonance, such that forces and pressures can be detected, it may be necessary or desirable to perform one or more calibration steps in advance of actuator operation, in order to determine the resonance or anti-resonance frequency of the device. To this end a 'sweep' may be performed, for each of two or more fixed actuation voltages, across a range of sensor-signal frequencies, and a corresponding series resistance measured for each of the sensor frequencies.

Figure 7 illustrates a set of results for one example sweep, wherein measured series resistance (in Ohms) is shown on the y-axis 104, and sensor signal frequency (in Hz) on the x-axis 106, and wherein plot 108 shows the corresponding trace for an actuation voltage of 0V (i.e. no actuation) and plot 110 the trace for an actuation voltage of 150V. As can be seen from the graph, the resistance values for the 150V sweep demonstrate a slight jump at two points along the sweep - at around 24 KHz and at around 40 KHz.

The resistance values for the 0V sweep indicate no variation about the primary curve (which reflects simply a capacitive complex impedance function) as the AC frequency is varied. The efficiency of the electromechanical coupling in the EAP material is dependent on the magnitude of the DC bias voltage (the greater the DC bias, the better the coupling). At 0V bias, there is little or no coupling, and hence zero (or immeasurably small) deformation response in the material to the AC signal. The 0V bias sweep hence provides a convenient baseline against which to compare an AC frequency sweep at a higher (actuation inducing) DC voltage.

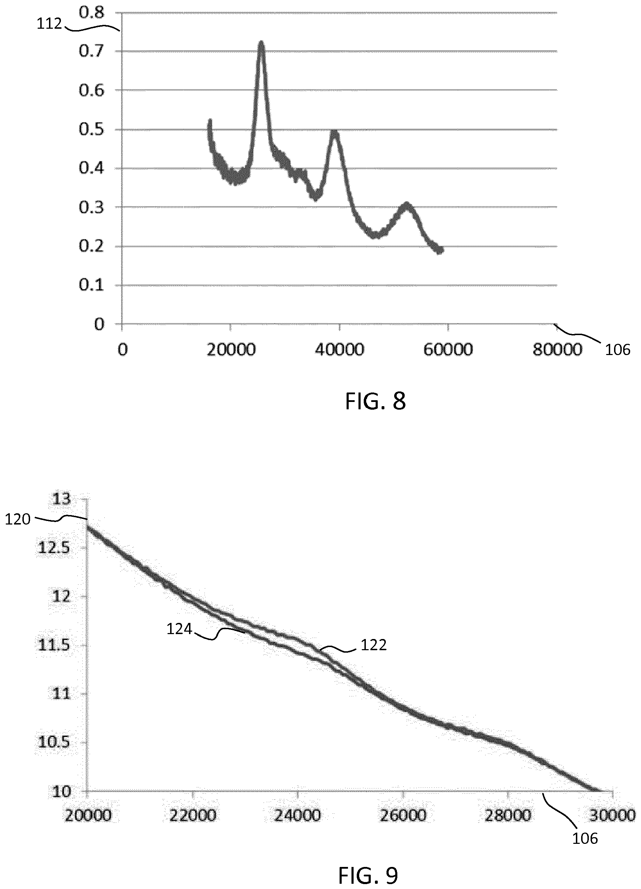

The anti-resonance frequency of the device may be identified by finding the AC frequency for which the difference between the measured resistance values for the two DC voltages is the greatest. In Figure 8 is illustrated more clearly the differences between the two signal traces 108 and 110, with difference in measured resistance 112 on the y-axis and corresponding sensor signal frequency 106 on the x-axis. The two larger jumps in resistance are clearly visible in this graph, with the larger of the two being the jump occurring at 24 KHz. Hence the anti-resonance frequency for the example device represented by Figures 7 and 8 is 24 KHz. This is the point of highest sensitivity for the device, i.e. the point at which the series resistance is most sensitive to changes in the frequency of the applied drive signal (or to changes in the anti-resonance frequency of the structure, for a fixed applied drive frequency).

Although a DC bias of 0V is used for the first sweep in the example of Figures 7 and 8, in alternative examples a different (non-zero) first bias might be used. In this case, depending on the magnitude of the first voltage, the first sweep may indicate variations or peaks about the central curve. However, the anti-resonance frequency may still be found by identifying the frequency for which the difference between the measured resistance values for the two DC voltages is the greatest.

To illustrate the effect of applying a load to the device, Figure 9 shows two resistance 120 versus frequency 106 'sweeps' for the same fixed (150V) DC bias voltage, but corresponding to differing loads applied to the actuator.

Line 122 represents the sweep for no load applied to the device. This line is hence identical to line 110 in Figure 7, but shown for a narrower range of frequencies and resistances. Line 124 represents the sweep for a load of 0.01N applied to the actuator. As can be seen, the effect of the load is to effectively 'iron out' the bump in resistance at the device resonance frequency of ~24 KHz. The application of 0.01N to the device is enough to dampen out much of the resonance effect caused by the applied high frequency signal. This dampening out allows the presence of even small loads to be detected.

This dampening effect is greater the higher the magnitude of applied load force. This relationship allows applied loads not just to be detected, but also to be measured quantitatively. To achieve measuring of loads, it may be necessary to perform an additional calibration step in advance of operation of the actuator. This calibration step is performed after determination of the anti-resonance frequency (described above). Once the anti-resonance frequency is known, a sweep may be performed, for fixed DC bias voltage, and for fixed AC frequency (i.e. the anti-resonance frequency), but measuring series resistance as a function of applied load to the device. Once this relationship is known, for a given fixed frequency signal, it may be utilised while the device is in operation to allow measured series resistance to provide a near direct measure of the magnitude of applied load.

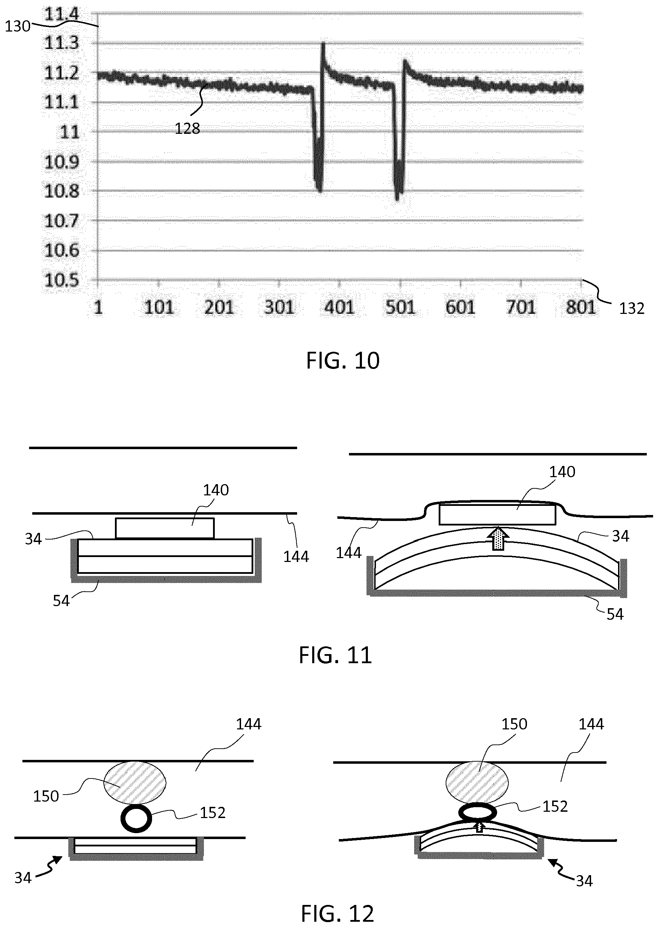

To illustrate this, in Figure 10 is shown a signal 128 representing the measured series resistance 130 (in Ohms) over time (in arbitrary units) 132 for an example actuator device being driven at a fixed DC bias of 150V and at a fixed AC frequency of 24KHz (the resonant frequency of the device in question). At times t=350 and time t=500, the actuator is loaded with a 0.01N load. This leads in each case to a sharp decline in resistance 130, which lasts for the duration of each applied load. It is clear from Figure 10 that the device provides a fast and highly precise response to applied loads, which is ideal for sensor applications. Although the magnitude of the applied force is already known in this case, through performing the calibration step described above in advance of operation, a graph of the sort shown in Figure 10 could readily be used to determine not just the timings of load events, but also their precise magnitudes.

As discussed above, a number of different configuration options are achievable in accordance with the present invention to provide auto-adjustment of the positioning of a physiological sensing surface against a human tissue receiving surface.

According to at least a first set of example embodiments, the EAP structure described above may be arranged and adapted to manipulate a sensing surface of a further additional physiological sensor device. This device is ideally integrated as a part of the sensing apparatus itself. An example is shown by way of illustration in Figure 11, wherein the sensor apparatus comprises a further physiological sensor element 140, having a sensing surface arranged to face an incident region of a surface of a subject's skin 144.

The sensor element 140 may be configured to provide physiological sensing functionality in accordance with any of a wide range of different principles or methods. These include, but are not limited to, devices to measure EEG, ECG, heartbeat, oxygen content in blood ($SPO_2$) and blood pressure. In one advantageous set of embodiments a PPG based sensing element may be integrated into the sensing apparatus. One or more optical sensing surfaces of such a PPG based device may be arranged cooperatively with the EAP structure to allow adjustment of the positioning of the surfaces relative to a surface of skin.

The sensing element may include a signal output (wired or non-wired) for outputting physiological sensing data.

As shown, the EAP structure 34 is provided clamped within a housing 54. The left side of Figure 11 shows the structure in an idle, unactuated state. The right side of Fig 11 shows the structure in an active, actuated state. Upon application of an actuation signal, the structure deforms upwards, thereby pressing the physiological sensing element 140 against the surface of the region of skin or tissue 144. Through application of the AC sensing signal and measurement of impedance or resistance values, a measure of the return force applied to the sensing element 140 by the skin receiving surface 144 may be obtained. Though applied to the sensor element, since the element is arranged in direct mechanical communication with the EAP structure, this force is transferred through the element, and is measurable by the EAP structure.

The sensed return force values are used by the controller (not shown) to regulate the magnitude of the applied actuation signal. For example the controller may be adapted to adjust the actuation signal so as to maintain the sensed impedance, resistance or force value substantially constant or uniform.

According to at least a second set of examples, the EAP structure itself may be adapted to provide measurements of one or more physiological parameters. In this case, a surface of the EAP structure itself provides the sensing surface which is manipulated by means of the apparatus in order to adjust its positioning against the human tissue receiving surface. Figure 12 shows one example, in which the EAP structure 34 is implemented to provided tonometry-based measurement of blood pressure.

The figure shows an example EAP structure 34 as applied against a surface 144 of a subject's skin, directly above an artery 152, the artery being aligned with a bone 150 located beneath the artery. The left side of the figure shows the structure in an idle un-actuated state. The right side shows the EAP structure in an active actuated state. As shown, activation of the EAP structure 34 applies pressure to the artery 152, thereby pressing it against the bone 150 positioned underneath. As pressure is applied to the skin surface 144 by the EAP structure, a returning force is exerted by the skin surface back toward the contacting surface of the EAP structure. This returning force may be sensed by means of application of a superposed resonant or anti-resonant AC sensing signal, and measurement of exhibited impedance or resistance values over time (as described above).

The obtained force or impedance values may typically be composed of two components. The first component is a reaction force component, generated in response to application of the actuating force, being roughly equal and opposite in magnitude and direction to the applied actuation force. This component arises as a result of Newton's third law and, since it is equal and opposite to the actuation force, may provide a proxy measure of the actuation force being applied to the skin by the EAP structure 34. The second component is a physiological component, arising as a result of the pressure of the blood flowing through the artery 152.

For the purposes of this embodiment, the controller (not shown) may comprise a filter circuit which includes one or both of a high-pass filter and low-pass filter. The filter circuit allows extraction from the obtained impedance or resistance values a subset of values representing each of the reaction force component and the physiological component. The low pass filter allows extraction of the reaction force component, since this component is typically relatively static, and exhibits little or no fluctuation. Its frequency is effectively close to zero. The high pass filter enables extraction of a physiological component, corresponding for example to blood pressure or pulse rate, since this naturally oscillates in accordance with the cyclical pulsing of blood through the artery.

As discussed in a preceding section, for tonometry it is important that the force applied to the artery is controlled carefully to provide at least partial compression of the artery, but not full occlusion. The integrated force sensing capability of the EAP structure 34 may be utilized to provide feedback on the magnitude of force being applied to the artery 152. In particular, the controller may be adapted to adjust the actuation voltage applied to the EAP structure in dependence upon the extracted reaction force component of the sensed returning force. The controller may be programmed for instance to ensure that the reaction force component remains within a certain set of defined threshold values.

Once a required pressure or force is achieved, the controller may be adapted to implement a control loop function for instance, whereby changes in sensed reaction force stimulate an appropriate adjustment of the applied actuation voltage in order to maintain the reaction force (and hence the applied actuation force) at the required level.

This functionality also enables the apparatus to compensate for any unintentional motion artifacts caused by accidental external impacts, or by movements of the user.

Where an appropriate filter circuit is included, this adjustment control loop may implemented at the same time as obtaining measurements of blood pressure of blood flowing through the artery 152.

To enhance efficiency or accuracy, according to a variation on this embodiment, a timing scheme may be implemented wherein physiological measurements and reaction force measurements are obtained at different times, in an alternating or sequential manner. Where physiological measurements are required, the control loop function may be ceased or paused while the impedance signal is analyzed to obtain these measures. Once physiological measures have been obtained, the control loop may be re-commenced. This would avoid the need to obtain both signals simultaneously, thus simplifying the control processes, and reducing necessary processing power for example.

In a further variation, the controller may be adapted to adjust the magnitude of the applied actuation voltage (and hence the actuation force) in dependence upon the physiological component of the measured returning force only. In particular, pressure variations caused by changes of the blood pressure in the artery 152 are compensated by adjustment of the actuator drive signal, so as to maintain a roughly uniform sensed returning force. Since only the physiological component is used for both controlling adjustment of the EAP actuation extent, and obtaining physiological measures, only a single filter (low pass filter) is required, and only one signal need be extracted. This reduces complexity of the control electronics, thus reducing cost and complexity of manufacture, and also limiting the required processing power, and processing complexity.

According to a further example set of embodiments, the sensing apparatus may be adapted for detection of oscillating blood pressure changes. In this case, the controller is adapted to control the EAP structure 34 to first apply an initial pressure to the skin surface 144 to thereby partially compress the artery 152 against the bone 150 beneath. The level of this initial pressure may be controlled by means of measurement of the reaction force component of the return force for instance. This pressure is then subsequently slowly reduced. During the reduction of the pressure, the physiological component of the sensed impedance, resistance or force values is monitored to detect and record the oscillating changes in impedance (exhibited force) occurring as a result of oscillating of the blood vessel walls, caused by blood flow between systolic and diastolic blood pressure.

In accordance with this or other tonometry-based embodiments, the controller may be adapted to adjust the actuation signal in dependence upon the sensed blood pressure (or other physiological parameter). For example, the controller may be adapted to detect changes in the sensed blood pressure and to compensate for these variations to ensure a constant contact force is maintained between the skin surface and the EAP surface. For instance, if the blood pressure gets higher, the EAP actuation force may be reduced (by lowering the actuation voltage), and vice versa. This may manifest in controlling the EAP actuation voltage so as to maintain steady detected impedance.

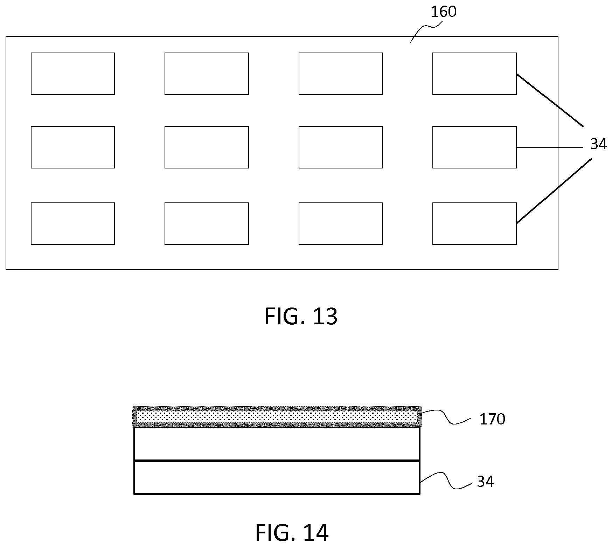

In accordance with a further set of embodiments, an array of EAP structures 34 may be provided as part of the sensor apparatus, mounted to a flexible carrier 160, wherein each EAP structure is controlled in accordance with the methods and principles described above. An example is shown in Figure 13.

Each structure 34 may be operable to provide independent measures of a particular physiological parameter at a different particular location of the user's skin. This improves accuracy and reliability of measures, and also allows further physiological information to be derived, such as regional variations in physiological parameters, or directional information.

In addition, such a plural arrangement may reduce the necessity of highly precise positioning of the EAP structures 34, since multiple different areas can be sensed at once, ensuring that data is not missed for instance.

The plurality of EAP structures 34 may be arranged in a two-dimensional configuration on the flexible carrier 160. This may form a regular array or an irregular array or formation.

EAPs are intrinsically flexible. This enables the structures to be readily integrated into a flexible carrier 160. The flexible carrier might be incorporated into a flexible band/bandage or other wearable structure, to provide a wearable physiological sensor apparatus. This could in examples be applied to e.g. the arm (e.g. wrist), a finger, or even embedded into a plaster-like adhesive which might be adhered to any desired part of the human body.

According to further example set of embodiments, additional force or pressure-sensing elements may be provided to improve or augment the sensing capability of the EAP structure. An example is shown in Figure 14. Here, the sensor apparatus further comprises a layer of piezoelectric material 170 in the form of an electrode, mechanically adhered to the electroactive polymer structure, and electrically coupled with the controller. The layer is operable to measure an applied force exerted upon it by the receiving surface. This may provide an additional means for measuring forces exerted by the receiving surface. This may improve sensitivity or provide further information which may assist in measuring a physiological parameter or in measuring a reaction force applied by the receiving surface.

The piezoelectric material 170 may be a layer of polyvinylidene fluoride (PVDF) material. This material can form a thin, highly flexible sheet or film which can be arbitrarily shaped and stacked to generate a multilayer configuration for example. This renders it highly compatible with incorporation onto an EAP structure 34 as shown in the example of Figure 14.

The PVDF electrode 170 may comprise more than one layer of PVDF. The electrode may be mechanically adhered (e.g. glued) to the EAP structure 34. The sensing output of the piezoelectric electrode may be utilised by the controller to regulate or adjust the actuation signal supplied to the EAP structure. The actuation signal may thereby be controlled at least partially in dependence upon an output of the piezoelectric layer 170. The piezoelectric layer/electrode may additionally or alternatively be utilised to sense or measure pressure applied as a result of one or more physiological parameters or phenomena, such blood pressure, or a pulse rate.

Other sensing elements may additionally or alternatively be incorporated, such as a strain gauge, or small solid pressure sensors such as pressure cells or integrated SMD components.

In accordance with further example embodiments, the EAP structure may be further controlled to provide temperature measurements in addition to load/pressure measurements. These can both be provided simultaneously with actuation. Temperature sensing can be directly achieved in embodiments through superposition of a second low-amplitude AC sensing signal atop the actuation signal, at a frequency which differs from the frequency of the first (primary) sensing signal (for sensing pressure/load).

The second frequency is a frequency for which the impedance or series resistance exhibited by the EAP structure is constant with respect to applied loads, but for which the impedance or series resistance varies in a predictable way with respect to temperature.

The first and second sensing signals may for example be applied alternatingly, to thereby decouple the influence of temperature on the pressure signal. The alternating application of the two sensing signals may be performed at a relatively fast alternation frequency, such that load/pressure measurements and temperature measurements are achieved substantially simultaneously. Signal application time is in this way effectively split between temperature and pressure/force sensing, but in such a way that measurements of each may still be obtained with a high degree regularity.

To obtain temperature measurements, the second (temperature) sensing signal must be at a frequency for which the exhibited impedance changes in a reliable and predictable manner in dependence upon the temperature of the EAP structure (or the surrounding environment of the EAP structure). The exhibited impedance at each given temperature will also in general depend upon the applied actuation signal at the time of measurement.

A calibration process may be performed in advance of operation, wherein impedance across the EAP structure is measured for each of an anticipated possible range of applied actuation signals, at each of a desired range of different temperatures. A look-up table of corresponding exhibited impedances for an applied actuation signal at each different temperature may accordingly be generated. This look-up table may be used during operation of embodiments to determine, based on a measured impedance and upon a known applied actuation signal, an estimated temperature of the EAP structure (or its environment).

Ascertainment of temperature measurements in addition to force/load measurements may provide further useful physiological information. Temperature measurements may enable further or more detailed or precise physiological information to be obtained. It may in examples be combined for instance with tonometric blood pressure measurements to add greater depth or detail to the pressure data.

In further examples, temperature measurement might be used as a means of determining or guiding positioning of the device relative to one or more physiological structures or features. This is particularly the case for physiological structures or features exhibiting a strong temperature characteristic, such as for instance veins or arteries, which typically exhibit an elevated temperature relative to surrounding tissue (due to the volume of blood flowing through them). This temperature 'signal' or indicator may be used in embodiments of the apparatus to enable determination of the position of the EAP structure relative to a given physiological feature, and for example to guide or inform either initial positioning of the EAP structure or actuated positional adjustments of the structure.

In preferred embodiments, the actuator layer may comprise a layer of relaxor ferroelectric material, which is particularly suitable for simultaneous sensing and actuation functions. Relaxor ferroelectric materials are non-ferroelectric when zero DC voltage is applied. Hence there is no electromechanical coupling present in the material. The electromechanical coupling becomes non-zero when a DC bias voltage is applied and can be measured through applying the small amplitude high frequency signal on top of the DC bias, in accordance with the procedures described above. Relaxor ferroelectric materials, moreover, benefit from a unique combination of high electromechanical coupling at non-zero DC bias and good actuation characteristics.

Suitable materials for the EAP layer include but are not limited to

Polyvinylidene fluoride - trifluoroethylene - chlorofluoroethylene (PVDF-TrFE-CFE), Polyvinylidene fluoride - trifluoroethylene - chlorotrifluoroethylene) (PVDF-TrFE-CTFE), Polyvinylidene fluoride- trifluoroethylene - hexafluoropropylene (PVDF - TrFE - HFP), or blends thereof.

Additional passive layers may be provided for influencing the behavior of the EAP layer in response to an applied electric field.

The EAP layer may be sandwiched between electrodes. The electrodes may be stretchable so that they follow the deformation of the EAP material layer. Materials suitable for the electrodes are also known, and may for example be selected from the group consisting of thin metal films, such as gold, copper, or aluminum or organic conductors such as carbon black, carbon nanotubes, graphene, poly-aniline (PANI), poly(3,4-ethylenedioxythiophene) (PEDOT), e.g. poly(3,4-ethylenedioxythiophene) poly(styrenesulfonate) (PEDOT:PSS). Metalized polyester films may also be used, such as metalized polyethylene terephthalate (PET), for example using an aluminum coating.

The materials for the different layers will be selected for example taking account of the elastic moduli (Young's moduli) of the different layers.

Additional layers to those discussed above may be used to adapt the electrical or mechanical behavior of the device, such as additional polymer layers.

The device may be used as a single actuator, or else there may be a line or array of the devices, for example to provide control of a 2D or 3D contour.

The invention can be applied in many EAP physiological sensor applications, including examples where a passive matrix array of actuators is of interest. For such applications, low voltage operation is desired, and the actuation voltage is preferably below 300V. The smaller AC sensing voltage may typically be below 20V.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform at the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

**Claims**

1. A physiological sensor apparatus for measuring at least one physiological parameter, and for controlling the positioning of a sensing surface against a human tissue receiving surface, comprising:

   said sensing surface;
   an electroactive polymer structure (34) adapted to deform in response to an application of an electrical signal, to thereby manipulate a positioning of the sensing surface; and
   a controller (42), adapted to:

   provide an electrical signal composed of superposed actuation signal and AC sensing signal to the electroactive polymer structure (34), the actuation signal for stimulating a deformation of the electroactive polymer structure (34) to thereby manipulate the sensing surface to apply an actuation force to the receiving surface, and the AC sensing signal for facilitating pressure sensing and having an AC frequency belonging to a harmonic of either a resonance or anti-resonance frequency of the electroactive polymer structure (34);
   monitor an impedance exhibited by the electroactive polymer structure (34) over time, to thereby provide an indication of a returning force exerted on the electroactive polymer structure by the receiving surface over time, and
   adjust a magnitude of the applied actuation signal in dependence upon the measured impedance and/or the returning force to thereby adjust the positioning of the sensing surface against the receiving surface.

2. The sensor apparatus as claimed in claim 1, wherein the controller (42) is adapted to adjust the magnitude of the actuation signal so as to maintain a steady actuation force applied against the receiving surface and/or so as to maintain a steady returning force, or component thereof, applied against the sensing surface.

3. The sensor apparatus as claimed in claim 1 or 2, wherein the controller (42) is adapted to adjust the magnitude of the actuation signal so as to maintain a steady relative distance between the sensing surface and the receiving surface and/or a point or body beneath the receiving surface.

4. The sensor apparatus as claimed in any preceding claim, wherein the controller (42) is adapted to decrease the magnitude of the actuation signal in response to falling or rising impedance values, and/or to increase the magnitude of the actuation signal in response to rising or falling impedance values.

5. The sensor apparatus as claimed in any preceding claim, wherein the controller (42) is adapted to decrease the magnitude of the actuation signal in response to impedance values falling below or rising above a defined threshold, and/or to increase the magnitude of the actuation signal in response to impedance values rising above or falling below a defined threshold.

6. The sensor apparatus as claimed in any preceding claim, further comprising a filter circuit adapted to filter the obtained impedance values so as to extract a reaction force component, representing forces exerted by an intrinsic elasticity of the receiving surface, and/or a physiological component, representing forces exerted as a result of one or more physiological phenomena, and optionally wherein
the physiological component represents forces associated with blood pressure and/or a cardiovascular pulse wave.

7. The sensor apparatus as claimed in claims 6, wherein the controller (42) is adapted to adjust the magnitude of the actuation signal in dependence upon either the reaction force component or the physiological component of the measured impedance values.

8. The sensor apparatus as claimed in any preceding claim wherein
the sensing surface is a surface of the electroactive polymer structure (34), or
the sensing surface is a surface of a further sensing component (140), arranged in mechanical co-operation with the electroactive polymer structure (34), and adapted to measure one or more physiological parameters.

9. The sensor apparatus as claimed in any preceding claim, wherein the controller (42) is adapted to apply a actuation signal which steadily decreases in magnitude over a defined time period, and is further adapted to process the measured impedance values over said time period to detect and measure oscillatory changes in the value over time, these changes being indicative of oscillating blood-vessel walls caused by blood pressure.

10. The sensor apparatus as claimed in any preceding claim, wherein the apparatus comprises an array of electroactive polymer structures (34), each independently controllable by the controller (42) to manipulate a respective sensing surface to apply a force at a respective point on the receiving surface and to measure a returning force exerted by the receiving surface at said point.

11. The sensor apparatus as claimed in any preceding claim, wherein the electroactive polymer structure (34) and/or the controller are mounted to a flexible carrier (160) for application to a region of the receiving surface.

12. The sensor apparatus as claimed in any preceding claim, further comprising a layer of piezoelectric material (170) mechanically adhered to the electroactive polymer structure and/or to the receiving surface, and electrically coupled with the controller, the layer being operable to measure an applied force exerted upon it by the receiving surface.

13. The sensor apparatus as claimed in any preceding claim, wherein the electroactive polymer structure (34) comprises a relaxor ferroelectric polymer.

14. The sensor apparatus as claimed in any preceding claim, wherein a magnitude of the sensing signal is smaller than 10 percent, preferably smaller than 1 percent, of a magnitude of the actuation signal.

15. A method of adjusting a physiological sensing apparatus to optimise a positioning of a sensing surface against a human tissue receiving surface, the apparatus comprising:

said sensing surface;
an electroactive polymer structure adapted to deform in response to the application of an electrical signal,
the method comprising:
providing apply an electrical signal composed of superposed actuation signal and AC sensing signal to the electroactive polymer structure, the actuation signal for stimulating a deformation of the electroactive polymer structure to thereby manipulate the sensing surface to apply an actuation force to the receiving surface, and the AC sensing signal for facilitating pressure sensing and having an AC frequency belonging to a harmonic of either a resonance or anti-resonance frequency of the electroactive polymer structure;
monitoring an impedance exhibited by the electroactive polymer structure over time, to thereby provide an indication of a returning force exerted on the electroactive polymer structure by the receiving surface over time, and
adjusting a magnitude of the applied actuation signal in dependence upon the measured impedance to thereby adjust the positioning of the sensing surface against the receiving surface.

16. A computer program product comprising a computer readable medium having computer readable code embodied therein, the computer readable code being configured such that, on execution by a suitable computer or processing unit, the computer or processing unit is caused to perform the method of claim 15.

**Patentansprüche**

1. Physiologische Sensorvorrichtung zur Messung von mindestens einem physiologischen Parameter und zum Steuern der Positionierung von einer Erfassungsoberfläche auf einer Aufnahmeoberfläche aus menschlichem Gewebe, umfassend:

die genannte Erfassungsoberfläche;
eine elektroaktive Polymerstruktur (34), die dafür ausgelegt ist, sich in Reaktion auf das Anlegen eines elektrischen Signals zu verformen, um dadurch eine Positionierung der Erfassungsoberfläche zu manipulieren; und
eine Steuereinheit (42), die ausgelegt ist zum:

Bereitstellen eines aus einem überlagerten Betätigungssignal und einem AC-Erfassungssignal zusammengesetzten elektrischen Signals für die elektroaktive Polymerstruktur (34), wobei das Betätigungssignal zum Stimulieren einer Verformung der elektroaktiven Polymerstruktur (34) dient, um dadurch die Erfassungsoberfläche zu manipulieren, um eine Betätigungskraft auf die Aufnahmeoberfläche auszuüben, und wobei das AC-Erfassungssignal zum Ermöglichen der Druckerfassung dient und eine AC-Frequenz aufweist, die zu einer Harmonischen von entweder einer Resonanz- oder Anti-ResonanzFrequenz der elektroaktiven Polymerstruktur (34) gehört;

Überwachen einer durch die elektroaktive Polymerstruktur (34) gezeigten Impedanz im Zeitverlauf, um dadurch eine Angabe einer Rückstellkraft bereitzustellen, die durch die Aufnahmeoberfläche im Zeitverlauf auf die elektroaktive Polymerstruktur ausgeübt wird, und

Anpassen einer Magnitude des angelegten Betätigungssignals in Abhängigkeit von der gemessenen Impedanz und/oder der Rückstellkraft, um dadurch die Positionierung der Erfassungsoberfläche auf der Aufnahmeoberfläche anzupassen.

2. Sensorvorrichtung nach Anspruch 1, wobei die Steuereinheit (42) dafür ausgelegt ist, die Magnitude des Betätigungssignals anzupassen, um eine konstante Betätigungskraft, die auf die Aufnahmeoberfläche ausgeübt wird, aufrechtzuerhalten und/oder um eine konstante Rückstellkraft oder Komponente hiervor, die auf die Erfassungsoberfläche ausgeübt wird, aufrechtzuerhalten.

3. Sensorvorrichtung nach Anspruch 1 oder 2, wobei die Steuereinheit (42) dafür ausgelegt ist, die Magnitude des Betätigungssignals anzupassen, um so einen konstanten relativen Abstand zwischen der Erfassungsoberfläche und der Aufnahmeoberfläche und/oder einem Punkt oder Körper unterhalb der Aufnahmeoberfläche aufrechtzuerhalten.

4. Sensorvorrichtung nach einem der vorhergehenden Ansprüche, wobei die Steuereinheit (42) dafür ausgelegt ist, die Magnitude des Betätigungssignals in Reaktion auf das Fallen oder Steigen von Impedanzwerten zu verringern und/oder um die Magnitude des Betätigungssignals in Reaktion auf das Steigen oder Fallen von Impedanzwerten zu erhöhen.

5. Sensorvorrichtung nach einem der vorhergehenden Ansprüche, wobei die Steuereinheit (42) dafür ausgelegt ist, die Magnitude des Betätigungssignals in Reaktion darauf zu verringern, dass Impedanzwerte unter einen definierten Schwellenwert fallen oder über einen definierten Schwellenwert ansteigen, und/oder um die Magnitude des Betätigungssignals in Reaktion darauf zu erhöhen, dass Impedanzwerte über einen definierten Schwellenwert ansteigen oder unter einen definierten Schwellenwert fallen.

6. Sensorvorrichtung nach einem der vorhergehenden Ansprüche, ferner umfassend eine Filterschaltung, die dafür ausgelegt ist, die erlangten Impedanzwerte zu filtern, um eine Reaktionskraftkomponente zu extrahieren, die die durch eine intrinsische Elastizität der Aufnahmeoberfläche ausgeübten Kräfte darstellt, und/oder eine physiologische Komponente zu extrahieren, die die infolge von einem oder mehreren physiologischen Phänomenen ausgeübten Kräfte darstellt, und wobei optional

die physiologische Komponente Kräfte darstellt, die dem Blutdruck und/oder einer kardiovaskulären Pulswelle zugehörig sind.

7. Sensorvorrichtung nach Anspruch 6, wobei die Steuereinheit (42) dafür ausgelegt ist, die Magnitude des Betätigungssignals in Abhängigkeit von entweder der Reaktionskraftkomponente oder der physiologischen Komponente der gemessenen Impedanzwerte anzupassen.

8. Sensorvorrichtung nach einem der vorhergehenden Ansprüche, wobei

die Erfassungsoberfläche eine Oberfläche der elektroaktiven Polymerstruktur (34) ist, oder

die Erfassungsoberfläche eine Oberfläche von einer weiteren Erfassungskomponente (140) ist, die in mechanischer Zusammenwirkung mit der elektroaktiven Polymerstruktur (34) angeordnet ist und dafür ausgelegt ist, einen oder mehrere physiologische Parameter zu messen.

9. Sensorvorrichtung nach einem der vorhergehenden Ansprüche, wobei die Steuereinheit (42) dafür ausgelegt ist, ein Betätigungssignal anzulegen, dessen Magnitude über einen definierten Zeitraum stetig abnimmt, und ferner dafür ausgelegt ist, die gemessenen Impedanzwerte über den genannten Zeitraum zu verarbeiten, um oszillierende Änderungen im Wert über die Zeit zu detektieren und zu messen, wobei diese Änderungen auf das Oszillieren von Blutgefäßwänden aufgrund des Blutdrucks hinweisen.

10. Sensorvorrichtung nach einem der vorhergehenden Ansprüche, wobei die Vorrichtung eine Anordnung aus elektroaktiven Polymerstrukturen (34) umfasst, die jede unabhängig durch die Steuereinheit (42) steuerbar sind, um eine jeweilige Erfassungsoberfläche zu manipulieren, um eine Kraft an einen jeweiligen Punkt auf der Aufnahmeoberfläche auszuüben und eine durch die Aufnahmeoberfläche an dem genannten Punkt ausgeübte Rückstellkraft zu messen.

**11.** Sensorvorrichtung nach einem der vorhergehenden Ansprüche, wobei die elektroaktive Polymerstruktur (34) und/oder die Steuereinheit auf einem flexiblen Träger (160) zur Anwendung auf eine Region der Aufnahmeoberfläche montiert sind.

**12.** Sensorvorrichtung nach einem der vorhergehenden Ansprüche, ferner umfassend eine Schicht aus piezoelektrischem Material (170), die mechanisch auf die elektroaktive Polymerstruktur und/oder die Aufnahmeoberfläche aufgebracht ist und elektrisch mit der Steuereinheit gekoppelt ist, wobei die Schicht dazu dient, eine ausgeübte Kraft zu messen, die durch die Aufnahmeoberfläche darauf ausgeübt wird.

**13.** Sensorvorrichtung nach einem der vorhergehenden Ansprüche, wobei die elektroaktive Polymerstruktur (34) ein ferroelektrisches Relaxor-Polymer umfasst.

**14.** Sensorvorrichtung nach einem der vorhergehenden Ansprüche, wobei eine Magnitude des Erfassungssignals kleiner als 10 Prozent, vorzugsweise kleiner als 1 Prozent, einer Magnitude des Betätigungssignals ist.

**15.** Verfahren zum Anpassen einer physiologischen Erfassungsvorrichtung zur Optimierung einer Positionierung von einer Erfassungsoberfläche auf einer Aufnahmeoberfläche aus menschlichem Gewebe, wobei die Vorrichtung umfassend:

die genannte Erfassungsoberfläche;
eine elektroaktive Polymerstruktur, die dafür ausgelegt ist, sich in Reaktion auf das Anlegen eines elektrischen Signals zu verformen,
wobei das Verfahren Folgendes umfasst:

Bereitstellen des Anlegens eines aus einem überlagerten Betätigungssignal und einem AC-Erfassungssignal zusammengesetzten elektrischen Signals an die elektroaktive Polymerstruktur, wobei das Betätigungssignal zum Stimulieren einer Verformung der elektroaktiven Polymerstruktur dient, um dadurch die Erfassungsoberfläche zu manipulieren, um eine Betätigungskraft auf die Aufnahmeoberfläche auszuüben, und wobei das AC-Erfassungssignal zum Ermöglichen der Druckerfassung dient und eine AC-Frequenz aufweist, die zu einer Harmonischen von entweder einer Resonanz- oder Anti-ResonanzFrequenz der elektroaktiven Polymerstruktur gehört;
Überwachen einer durch die elektroaktive Polymerstruktur (34) gezeigten Impedanz im Zeitverlauf, um dadurch eine Angabe einer Rückstellkraft bereitzustellen, die durch die Aufnahmeoberfläche im Zeitverlauf auf die elektroaktive Polymerstruktur ausgeübt wird, und
Anpassen einer Magnitude des angelegten Betätigungssignals in Abhängigkeit von der gemessenen Impedanz, um dadurch die Positionierung der Erfassungsoberfläche auf der Aufnahmeoberfläche anzupassen.

**16.** Computerprogrammprodukt umfassend ein computerlesbares Medium mit darauf verkörpertem computerlesbaren Code, wobei der computerlesbare Code derartig konfiguriert ist, dass er, wenn er durch einen geeigneten Computer oder eine geeignete Verarbeitungseinheit ausgeführt wird, den Computer oder die Verarbeitungseinheit veranlasst, das Verfahren nach Anspruch 15 durchzuführen.

**Revendications**

**1.** Appareil de détection physiologique destiné à la mesure d'au moins un paramètre physiologique, et à la commande du positionnement d'une surface de détection contre une surface réceptrice d'un tissu humain, comprenant :

ladite surface de détection;
une structure de polymère électroactif (34) adaptée pour se déformer en réponse à une application d'un signal électrique, pour ainsi manipuler un positionnement de la surface de détection ; et
une unité de commande (42), adaptée pour :

fournir un signal électrique composé d'un signal d'actionnement et d'un signal de détection AC superposés à la structure de polymère électroactif (34), le signal d'actionnement pour stimuler une déformation de la structure de polymère électroactif (34) pour ainsi manipuler la surface de détection afin qu'elle applique une force d'actionnement sur la surface réceptrice, et le signal de détection AC pour faciliter la détection de la pression et présentant une fréquence AC appartenant à une harmonique de soit une fréquence de résonnance, soit une fréquence anti-résonnance de la structure de polymère électroactif (34) ;

surveiller une impédance exposée par la structure de polymère électroactif (34) au fil du temps, pour ainsi fournir une indication d'une force de retour exercée sur la structure de polymère électroactif par la surface réceptrice au fil du temps, et

ajuster une magnitude du signal d'actionnement appliqué en fonction de l'impédance mesurée et/ou la force de retour pour ainsi ajuster le positionnement de la surface de détection contre la surface réceptrice.

2. Appareil de détection physiologique selon la revendication 1, dans lequel le dispositif de commande (42) est adapté pour ajuster la magnitude du signal d'actionnement de façon à maintenir une force d'actionnement régulière appliquée contre la surface réceptrice et/ou de façon à maintenir une force de retour régulière, ou un composant de celle-ci, appliqué(e) contre la surface de détection.

3. Appareil de détection selon la revendication 1 ou 2, dans lequel le dispositif de commande (42) est adapté pour ajuster la magnitude du signal d'actionnement de façon à maintenir une distance relative régulière entre la surface de détection et la surface réceptrice et/ou un point ou corps en dessous de la surface réceptrice.

4. Appareil de détection selon l'une quelconque des revendications précédentes, dans lequel le dispositif de commande (42) est adapté pour diminuer la magnitude du signal d'actionnement en réponse à des valeurs d'impédance en augmentation ou en diminution, et/ou pour augmenter la magnitude du signal d'actionnement en réponse à des valeurs d'impédance en augmentation ou en diminution.

5. Appareil de détection selon l'une quelconque des revendications précédentes, dans lequel le dispositif de commande (42) est adapté pour diminuer la magnitude du signal d'actionnement en réponse à des valeurs d'impédance diminuant en dessous ou augmentant au-dessus d'un seuil défini, et/ou pour augmenter la magnitude du signal d'actionnement en réponse à des valeurs d'impédance augmentant au-dessus ou diminuant en dessous d'un seuil défini.

6. Appareil de détection selon l'une quelconque des revendications précédentes, comprenant en outre un circuit de filtration adapté pour filtrer les valeurs d'impédance obtenues de façon à extraire un composant de force de réaction, représentant des forces exercées par une élasticité intrinsèque de la surface réceptrice, et/ou un composant physiologique, représentant ses forces exercées en résultat d'un ou plusieurs phénomènes physiologiques, et facultativement dans lequel

le composant physiologique représente des forces associées à la pression sanguine et/ou à une onde de pulsation cardiovasculaire.

7. Appareil de détection selon la revendication 6, dans lequel le dispositif de commande (42) est adapté pour ajuster la magnitude du signal d'actionnement en fonction soit du composant de force de réaction, soit du composant physiologique des valeurs d'impédance mesurées.

8. Appareil de détection selon l'une quelconque des revendications précédentes dans lequel

la surface de détection est une surface de la structure de polymère électroactif (34), ou

la surface de détection est une surface d'un autre composant de détection (140) agencée en coopération mécanique avec la structure de polymère électroactif (34), et adaptée pour mesure un ou plusieurs paramètres physiologiques.

9. Appareil de détection selon l'une quelconque des revendications précédentes, dans lequel le dispositif de commande (42) est adapté pour appliquer un signal d'actionnement dont la magnitude diminue de façon constante au fur et à mesure d'une période de temps définie pour détecter et mesurer les changements oscillatoires dans la valeur au fil du temps, ces changements étant indicateurs de l'oscillation des parois des vaisseaux sanguins causée par la pression sanguine.

10. Appareil de détection selon l'une quelconque des revendications précédentes, dans lequel l'appareil comprend un faisceau de structures de polymère électroactif (34), chacun pouvant être commandé indépendamment par le dispositif de commande (42) pour manipuler une surface de détection respective afin qu'elle applique une force à moment respectif sur la surface réceptrice et qu'elle mesure une force de retour exercée par la surface réceptrice audit moment.

11. Appareil de détection selon l'une quelconque des revendications précédentes, dans lequel la structure de polymère électroactif (34) et/ou le dispositif de commande sont montés sur un vecteur flexible (160) pour l'application dans une région de la surface réceptrice.

12. Appareil de détection selon l'une quelconque des revendications précédentes, comprenant en outre une couche de matériau piézoélectrique (170) qui adhère mécaniquement à la structure de polymère électroactif et/ou à la surface réceptrice, et qui est couplé électriquement au dispositif de commande, la couche pouvant être utilisée pour mesurer une force appliquée exercée sur elle par la surface réceptrice.

13. Appareil de détection selon l'une quelconque des revendications précédentes, dans lequel la structure de polymère électroactif (34) comprend un polymère ferroélectrique relaxeur.

14. Appareil de détection selon l'une quelconque des revendications précédentes, dans lequel une magnitude du signal de détection est inférieure à 10 pourcents, de préférence inférieure à 1 pourcent, d'une magnitude du signal d'actionnement.

15. Procédé d'ajustement d'un appareil de détection physiologique pour optimiser le positionnement d'une surface de détection contre une surface réceptrice d'un tissu humain, l'appareil comprenant :

   ladite surface de détection ;
   une structure de polymère électroactif adaptée pour se déformer en réponse à l'application d'un signal électrique ;
   le procédé comprenant les étapes consistant à :

   fournir l'application d'un signal électrique composé d'un signal d'actionnement et d'un signal de détection AC superposés à la structure de polymère électroactif, le signal d'actionnement pour stimuler une déformation de la structure de polymère électroactif pour ainsi manipuler la surface de détection afin qu'elle applique une force d'actionnement sur la surface réceptrice, et le signal de détection AC pour faciliter la détection de la pression et présentant une fréquence AC appartenant à une harmonique de soit une fréquence de résonnance, soit une fréquence anti-résonnance de la structure de polymère électroactif ;
   surveiller une impédance exposée par la structure de polymère électroactif (34) au fil du temps, pour ainsi fournir une indication d'une force de retour exercée sur la structure de polymère électroactif par la surface réceptrice au fil du temps, et
   ajuster une magnitude du signal d'actionnement appliqué en fonction de l'impédance mesurée et/ou la force de retour pour ainsi ajuster le positionnement de la surface de détection contre la surface réceptrice.

16. Produit de programme d'ordinateur comprenant un support lisible par ordinateur présentant un code lisible par ordinateur inscrit dans celui-ci, le code lisible par ordinateur étant configuré de sorte que, lors de son exécution par un ordinateur ou par une unité de traitement adaptée, l'ordinateur ou l'unité de traitement est incité(e) à effectuer le procédé selon la revendication 15.

FIG. 1

FIG. 2

FIG. 3

FIG. 4

FIG. 5

**FIG. 6**

**FIG. 7**

FIG. 8

FIG. 9

FIG. 10

FIG. 11

FIG. 12

160

34

FIG. 13

170

34

FIG. 14

**EP 3 515 286 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080033275 A
- WO 2016096391 A1